(19) Europäisches Patentamt
European Patent Office
Office européen des brevets

(11) Veröffentlichungsnummer: **0 127 099**
**B1**

(12) # EUROPÄISCHE PATENTSCHRIFT

(45) Veröffentlichungstag der Patentschrift: **20.05.87**

(51) Int. Cl.⁴: **C 07 D 209/88**, A 61 K 31/40

(21) Anmeldenummer: **84105747.4**

(22) Anmeldetag: **19.05.84**

(54) **Verfahren zur Herstellung von optisch aktiven Carbazol-Derivaten, neue R- und S-Carbazol-Derivate, sowie Arzneimittel, die diese Verbindungen enthalten.**

(30) Priorität: **26.05.83 DE 3319027**

(43) Veröffentlichungstag der Anmeldung:
**05.12.84 Patentblatt 84/49**

(45) Bekanntmachung des Hinweises auf die Patenterteilung:
**20.05.87 Patentblatt 87/21**

(84) Benannte Vertragsstaaten:
**AT BE CH DE FR GB IT LI LU NL SE**

(56) Entgegenhaltungen:
**EP-A-0 004 920**
**DE-B-2 240 599**

(73) Patentinhaber: **Boehringer Mannheim GmbH, Sandhofer Strasse 116, D-6800 Mannheim 31 (DE)**

(72) Erfinder: **Leinert, Herbert, Dr.- Ing., Essigkamm 11, D-6148 Heppenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 127 099 B1

LIBER, STOCKHOLM 1987

**Beschreibung**

Die Erfindung betrifft eine asymmetrische Synthese mit hoher optischer Reinheit von R- und S-Carbazol-Derivaten der Formel I

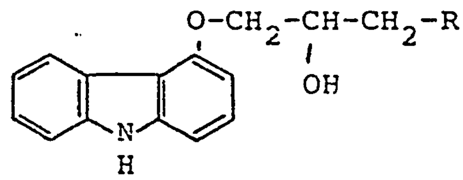

I,

in der R ein unsubstituierter oder substituierter Aminorest bedeutet, sowie deren pharmakologisch verträgliche Salze und ihre Verwendung als Arzneimittel.

R bedeutet insbesondere eine Aminogruppe, die durch eine niedere Alkylgruppe mit 1 - 6 C-Atomen, wie Methyl, Ethyl, Isopropyl oder tert. Butyl substituiert ist oder den Rest

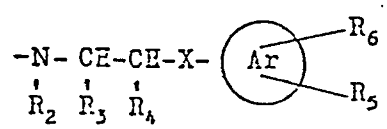

,

in dem
$R_2$ Wasserstoff, eine niedere Alkylgruppe oder eine Benzyl-, Phenylethyl- oder Phenylpropylgruppe,
$R_3$ Wasserstoff oder eine niedere Alkylgruppe,
$R_4$ Wasserstoff oder eine niedere Alkylgruppe,
X einen Valenzstrich, eine -$CH_2$-Gruppe, ein Sauerstoff- oder Schwefelatom,
Ar einen Phenyl-, Naphthyl-, Indanyl-, Tetrahydronaphthyl- oder Pyridyl-Rest und
$R_5$ und $R_6$, welche gleich oder verschieden sein können, jeweils Wasserstoff, Halogen, eine niedere Alkylgruppe, eine Aminocarbonylgruppe, eine Hydroxygruppe, eine niedere Alkoxygruppe, eine Benzyloxygruppe, eine niedere Alkylmercaptogruppe, eine niedere Alkylsulfinylgruppe, eine niedere Alkylsulfonylgruppe oder auch gemeinsam eine Methylendioxygruppe bedeuten.

Die niedrigen Alkylgruppen $R_2$, $R_3$, $R_4$, $R_5$ und $R_6$ und die niedere Alkoxy- niedere Alkylthio-, niedere Alkylsulfinylund niedere Alkylsulfonyl-Gruppen $R_5$ und $R_6$ haben 1 - 6, insbesondere 1 - 4 C-Atome.

Verbindungen mit den oben genannten Substituenten R sind in der DE-PS 22 40 599 und in der EP-PS 4 920 beschrieben.

Nach den in diesen Schriften aufgeführten Verfahren werden in allen Fällen Racemate der beschriebenen Verbindungen erhalten. Eine Trennung des Racemates in die optisch aktiven Antipoden geschieht nach an sich bekannten Methoden über die Diastereomeren-Spaltung unter Verwendung bekannter optisch aktiver Säuren oder Basen. Dieses Verfahren ist sehr aufwendig und liefert in der Regel keine reinen optisch aktiven Substanzen. Die Verunreinigungen durch den jeweiligen anderen Antipoden sind praktisch nicht zu vermeiden. Es stellte sich daher die Aufgabe, einen Syntheseweg für die Herstellung der Antipoden in reiner Form zu finden.

Diese Aufgabe wurde durch die vorliegende Erfindung gelöst. Zur Herstellung von R-Carbazol-Derivaten der Formel I wird ein S-Epoxid der Formel II

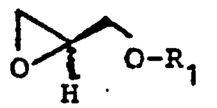

II,

in der $R_1$ ein substituiertes Sulfonsäure-Derivat bedeutet,

mit 4-Hydroxy-carbazol in Gegenwart eines organischen Lösungsmittels in alkalischem Medium umgesetzt und das erhaltene

R-4-(2,3-Epoxy-propoxy)-carbazol

mit Ammoniak oder einem substituierten Amin der oben angegebenen Bedeutung RH umgesetzt und anschließend die erhaltene Verbindung gewünschtenfalls in ein pharmakologisch verträgliches Salz überführt.

Substituierte Sulfonsäuren hinsichtlich $R_1$ sind vorzugsweise Methansulfonsäure p-Toluolsulfonsäure und Benzolsulfonsäure.

Das entsprechende S-Carbazol-Derivat der Formel I wird in ähnlicher Weise erhalten. Hierzu wird zunächst

R-(-)Epichlorhydrin mit 4-Hydroxy-carbazol in Gegenwart eines organischen Lösungsmittels in alkalischem Medium umgesetzt und das erhaltene

S-4-(2,3-Epoxy-propoxy)-carbazol mit Ammoniak oder einem substituierten Amin der oben genannten Bedeutung RH umgesetzt und anschließend die erhaltene Verbindung gewünschtenfalls in ein pharmakologisch verträgliches Salz überführt.

Die Herstellung der Schlüsselsubstanzen der Formel II, vorzugsweise des Mesyl-Derivates, und des R-(-)-Epichlorhydrin sind in der Literaturstelle Baldwin, J.org.Chem. Vol. 43, 1978, Seite 4876 beschrieben. Danach wird D-Mannit mit Aceton in Gegenwart von $ZnCl_2$ in 1,2,5,6-Di-o-isopropyliden-D-mannit überführt. Hierauf wird durch Spaltung mit Natriummetaperjodat und nachfolgender sofortiger Reduktion der intermediär entstehenden Aldehydfunktiondas S (+)-Isopropyliden-glycerin erhalten. Tosylierung dieser Substanz ergibt das R-3-Tosyloxy-propandiol-acetonid, das sofort ohne Isolierung in R (-)-3-Tosyloxy-1,2-propandiol überführt wird. Aus diesem wird durch Umsetzung mit Natriummethylat R-Glycidol erhalten, das wegen der Gefahr der Racemisierung sofort mit Methansulfonylchlorid in S (+)-3-Mesyloxy-1,2-epoxypropan überführt wird.

Zur Darstellung des R (-)-Epichlorhydrin wird S (+)-3-Mesyl-oxy-1,2-epoxypropan mit Salzsäure zu R-1-Chlor-2-hydroxy-3-mesyloxypropan geöffnet, das ohne Reinigung in Ethylenglykol mit Natriummethylenglykolat zu R (-)-Epichlorhydrin umgesetzt wird.

Die beiden aufgeführten Schlüsselsubstanzen werden jeweils mit 4-Hydroxy-carbazol unter Umkehr der Konfiguration zu den bisher nicht bekannten R (-)-4-(2,3-Epoxy-propoxy)-carbazol bzw. S (+)-4-(2,3-Epoxy-propoxy)-carbazol umgesetzt, die ebenfalls Gegenstand der Erfindung sind. Die beiden neuen Antipoden fallen bei den Verfahren mit einer optischen Reinheit von nahezu 100 % an.

Die optisch aktiven Antipoden des 4-(2,3-Epoxy-propoxy)-carbazols werden unter Beibehaltung der Konfiguration mit entsprechenden Aminen zu den optisch aktiven Verbindungen der Formel I umgesetzt. Hierzu wird in der Regel in einem organischen Lösungsmittel wie z.B. Methanol, Ethanol, Isopropanol, das Carbazol-Derivat mit dem Amin längere Zeit am Rückfluß erhitzt.

Die optisch aktiven Carbazol-Derivate der Formel I sind neue Verbindungen. Die pharmakologische Wirksamkeit der jeweiligen Antipoden ist im Vergleich zu der des Racemats sehr unterschiedlich. Während z.B. beim Carvedilol nur das S(-)-[1-Carbazolyl-(4)-oxy]-3-[2-(2-methoxy-phenoxy)]-ethylamino-propanol-(2) (linksdrehende Isomere - Bsp. 8) β-blockierende Eigenschaften aufweist, ist die vasodilatierende Wirkung bei beiden Isomeren dieser Verbindung vorhanden (vgl. Versuchsbericht). Aufgrund dieser Tatsache werden die unterschiedlichen pharmakologischen Eigenschaften bei der Entwicklung von Arzneimitteln nutzbar gemacht.

Durch freiwählbare Mischungsverhältnisse aus R- und S-Enantiomeren kann die jeweils günstigste Relation der beiden Wirkqualitäten zueinander gezielt eingestellt werden.

**Beispiel:**

Erweist sich bei einem Racemat die β-Blockade getragen durch S-Enantiomere, im Verhältnis zur Blutdrucksenkung getragen durch die R- und S-Enantiomeren, zu stark, kann man durch Veränderung des S-Anteils ein ausgewogeneres Wirkungsverhältnis erreichen.

Anwendbar sind demnach Mischungen von R: S von 1: 99 bis 99: 1. Ausgenommen im Sinne der Erfindung ist das Verhältnis 50: 50 (Racemat).

**Versuchsprotokoll**

**β-Blockade**

An wachen Kaninchen wurde die β-blockierende Wirkung anhand der Hemmung der Isoprenalintachycardie (entsprechend der Methode von Bartsch et al [Experiments in animals on the pharmacological effects of metipranolol in comparison with propranolol and pindolol - Drug Res. 27, (II), 12, 2319-2322, 1977]) bestimmt.

Als Maß für die β-blockierende Wirkstärke wurde die 50 %ige Hemmdosis berechnet.

**Vasodilation** (gemessen als unmittelbare Blutdrucksenkung nach einmaliger Applikation)

An wachenspontan hypertonen Ratten (SHR) wurden Katheter in die Arteria femoralis und die Vena jugularis implantiert. Über die Vene wurden entsprechende Dosen der Enantiomere (R-Carvedilol bzw. S-Carvedilol in den Dosen 0,03; 0,1; 0,3; 1,0 und 3 mg/kg i.v.) injiziert und über den arteriellen Druckkatheter (als Ausdruck der Vasodilatation) die arterielle Blutdrucksenkung erfaßt. Als Maß für den antihypertensiven Effekt wurden die Dosen zur Blutdrucksenkung um 30 mm Hg (= 4 kPa) berechnet.

**Wirkung von R- und S-Carvedilol (BM 14.190) auf Herz (M-Blockade) und Gefäße (Blutdruck)**

| | ß-Blockade | | Vasodilatation (= 4 kPa) | |
|---|---|---|---|---|
| (Reinheit %) | r | $ED_{50}$ % (mcg/kg i.v.) | r | ED-30 mm Hg (mcg/kg i.v.) |
| R-Carvedilol (> 99,4 %) | 0,96 | 3980 | 0,97 | 2960 |
| S-Carvedilol (> 99,4 %) | 0,99 | 25 | 0,96 | 270 |
| Relation R/S | - | 160 | - | 11 |

Cardevilol = [1-Carbazolyl-(4)-oxy]-3-[2-(2-methoxy-phenoxy)]-ethylamino-propanol-(2), Racemat

**Ergebnisse**

Die Resultate der Untersuchungen sowohl bezüglich der β-Blockade als auch der Blutdruck- bzw. Gefäßwirkung sind in der vorstehenden Tabelle dargestellt. Danach ergeben sich mit Korrelationskoeffizienten (r) von zwischen 0,96 bis 0,99 sehr gute Dosiswirkungsbeziehungen. Bezüglich der β-Blockade ist zwischen den beiden Enantiomeren ein so großer Unterschied, daß praktisch nur S-Carvedilol als β-Blocker bezeichnet werden kann. Erst in 160fach höherer Dosis ist auch für R-Carvedilol eine β-Blockade nachweisbar, die möglicherweise auf Spuren von S-Carvedilol zurückzuführen ist.

Bezüglich der blutdrucksenkenden Wirkung ist zwischen S- und R-Carvedilol ein vergleichsweise geringer Unterschied feststellbar. Der Differenzfaktor beträgt 11, die absolut erforderliche Dosis um eine Blutdrucksenkung um 30 mm Hg (= 4 kPa) zu erreichen, liegt bei S-Carvedilol mit 270 mcg/kg i.v. im Verhältnis zur β-Blockade ca. 10fach höher.

Eine weitere Besonderheit der unterschiedlichen pharmakologischen Eigenschaften von Enantiomeren einer Verbindung der Formel I ist, daß nur die R-Enantiomeren ein ausgeprägte Antiglaukomwirkung aufweisen und daher als optisch reine Substanzen bei der Glaukombehandlung Verwendung finden können.

Zur Überführung der Verbindungen der Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Phosphorsäure, Schwefelsäure, Essigsäure, Citronensäure, Maleinsäure, Benzoesäure um.

Zur Herstellung von Arzneimitteln werden die Verbindungen der Formel I in an sich bekannter Weise mit geeigneten pharmazeutischen Trägerstoffen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Zur Glaukombehandlung werden Verbindungen der Formel I bzw. ihre pharmakologisch unbedenklichen Salze in Form von Augentropfen verwendet. Bevorzugt sind Salze mit physiologisch verträglichen anorganischen oder organischen Säuren, z.B. Salzsäure, Bromwasserstoffsäure, Phosphorsäure,

Schwefelsäure, Essigsäure, Salicylsäure, Citronensäure, Benzoesäure, Naphthoesäure, o-Acetoxybenzoesäure, Adipinsäure oder Maleinsäure.

Geeignet sind isotone Lösungen mit einem pH von ca. 7,0. Als Medium kommt vorzugsweise Wasser zur Anwendung, welches übliche Zusätze wie Konservierungsmittel, Lösungsvermittler oder Puffer enthalten kann. Als Konservierungsstoffe kommen vorzugsweise Benzylalkohol, Benzalkoniumchlorid, Phenol oder Chlorhexidinacetat in Frage. Lösungsvermittler sind insbesondere Polyethylenglykole, Polyvinylpyrrolidon oder Glycerin. Als Puffer werden bevorzugt Essigsäure/Natriumacetat, Citronensäure/Natriumcitrat oder Natrium-EDTA verwendet.

Die erfindungsgemäßen Verbindungen der Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht-toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmelekulare Polymere (wie Polyethylenglykole), für orale Applikation geeignete Zubereitungen können gewünschtenfalls Geschmacksund Süßstoffe enthalten.

Die Erfindung wird durch die folgenden Beispiele näher erläutert:

**Beispiel 1**

**S (+)-3-Mesyloxy-1,2-epoxypropan**

10.5 g R-Glycidol werden in einer Mischung aus 23.3 ml Triethylamin und 210 ml absol. Toluol geloest. Hierzu tropft man bei 0-5°C unter Ruehren eine Loesung von 11.5 ml Methansulfonylchlorid in 50 ml absol. Toluol und laeßt dann ueber Nacht im Kuehlschrank stehen. Man saugt ab und dampft i. Vak. ein. Der Rueckstand wird in Methylenchlorid geloest, die Loesung mit 1 N HCl, gesaettigter Natriumhydrogenkarbonatloesung und Wasser gewaschen, ueber Natriumsulfat getrocknet und eingedampft. Der Rueckstand wird destilliert.

Ausbeute: 9 g Sdp: 100°C/0.8 mm (= 107 Pa)

$[\alpha]^{20}_D$: + 24 2° (c = 2.9; Methanol)

Das eingesetzte R-Glycidol wird wie folgt hergestellt:

a) <u>1,2,5,6-Di-o-isopropyliden-D-mannit</u>

Zu 2350 ml ueber neutralem Aluminiumoxid getrocknetes Aceton gibt man 200 ml Molekularsieb 3 Å, traegt langsam unter Ruehren 456 g Zinkchlorid ein, wobei sich die Loesung leicht erwaermt und laeßt dann ueber Nacht bei Raumtemperatur stehen. Anschließend traegt man unter Ruehren 285 g D (-)-Mannit ein und ruehrt noch 3 Std. bei Raumtemperatur weiter, wobei der Mannit in Loesung geht. Man saugt ab, waescht mit wenig trockenem Aceton den Filterrueckstand nach und gibt die Loesung sofort unter Ruehren auf eine Mischung aus 570 g Pottasche, 600 ml Wasser und 1700 ml Ether. Man filtriert vom ausgefallenen Zinkkarbonat ab und dampft ein. Der Rueckstand wird in Methylenchlorid aufgenommen und das noch vorhandene Wasser abgetrennt. Anschließend wird die Methylenchloridloesung ueber Natriumsulfat getrocknet, mit Floridin behandelt und weitgehend eingeengt. Dann gibt man 3 Liter Cyclohexan zu und laeßt kristallisieren. Der Rueckstand wird zur weiteren Reinigung nochmals aus Cyclohexan umkristallisiert.

Ausbeute: 200 g Schmp. 120-121°C

b) <u>S (+)-Isopropyliden-glycerin</u>

Zu einer Loesung von 199 g Natriummetaperjodat in 1680 ml Wasser gibt man unter Ruehren und Eiskuehlung im Verlauf von 45 min portionsweise 244 g 1,2,5,6-Di-o-isopropyliden-D-mannit. Nach beendeter Zugabe ruehrt man noch 15 min weiter und gibt dann 5 Liter Ethanol zu. Man saugt ab, waescht mit Ethanol nach und versetzt dann das Filtrat unter leichtem Kuehlen im Verlauf von 5 min mit 71 g Natriumborhydrid. Man ruehrt noch 2 Std. bei Raumtemperatur weiter, stellt dann den pH-Wert mit halbkonz. Essigsaeure auf 7.5 ein, laeßt noch 15 min stehen und saugt ab.

Der Filterrueckstand wird verworfen. Das Filtrat wird soweit eingedampft, bis kein Alkohol mehr uebergeht. Die zurueckbleibende waeßrige Loesung wird mehrmals mit Methylenchlorid extrahiert. Die vereinigten Methylenchloridphasen werden ueber Natriumsulfat getrocknet und eingedampft. Der Rueckstand wird ueber eine 40 cm Vigreux-Kolonne destilliert.

Ausbeute: 198.5 g; Sdp: 45°C/0.7 mm (= 93 Pa)

$[\alpha]_D^{20}$: + 11.6° (c = 10; Methanol)

$[\alpha]^{20}_D$. + 15.1° (c = 100)

c) <u>R (-)-3-Tosyloxy-1,2-propandiol</u>

Zu einer eiskalten Loesung von 36 g S (+)-Isopropylidenglycerin in 150 ml absol. Pyridin gibt man unter Ruehren portionsweise 52 g p-Toluolsulfonylchlorid. Nach beendeter Zugabe laeßt man ueber Nacht im Kuehlschrank stehen, verduennt dann die Loesung mit 150 ml Ether und waescht solange mit 1 N HCl, bis die waeßrige Phase einen sauren pH-Wert anzeigt. (Insgesamt etwa 600 ml 1 N HCl). Anschließend waescht man noch zweimal mit gesaettigter Natriumhydrogenkarbonatloesung, trocknet ueber Natriumsulfat, behandelt mit

Floridin und dampft ein. Man erhaelt 69.1 g eines oeligen Rueckstandes von R-3-Tosyloxy-propandiol-acetonid, der ohne weitere Reinigung weiter umgesetzt wird. Das Acetonid wird in einer Mischung aus 50 ml Aceton und 147 ml 1 N HCl 40 min auf 80°C erwaermt, wobei eine klare Loesung entsteht. Die Loesung wird i. Vak. eingedampft und der Rueckstand in Methylenchlorid geloest. Die Methylenchloridloesung wird ueber Natriumsulfat getrocknet und eingedampft. Der Rueckstand wird aus Diisopropylether umkristallisiert.

Ausbeute: 45 g Schmp: 62°C
$[\alpha]^{20}_D$: - 9.9° (c = 7.9; Methanol)
$[\alpha]^{20}_D$: - 6.8 (c = 7.5; Pyridin)

d) R-Glycidol

45 g R (-)-3-Tosyloxy-1,2-propandiol werden in einer Mischung aus 40 ml absol. Methanol und 75 ml absol. Ether geloest. Hierzu tropft man unter Ruehren bei 0-5°C innerhalb von 20 min eine Loesung von 4 g Natrium in 90 ml Methanol. Man ruehrt noch 2 Stdn. unter Kuehlung weiter, saugt ab, waescht mit Ether nach und dampft das Filtrat i. Vak. bei 20° Badtemperatur ein. Der Rueckstand wird nochmal in Ether aufgenommen, die Loesung mit Floridin behandelt, ueber Celite abgesaugt und eingedampft. Man erhaelt 10.5 g R-Glycidol als oeligen Rueckstand. (Dieser wird sofort weiter umgesetzt, um eine Racemisierung zu vermeiden.)

**Beispiel 2**

**R (-)-Epichlorhydrin**

Zu 32.7 g S (+)-3-Mesyloxy-1.2-epoxypropan tropft man unter guter Kuehlung 130 ml konz. Salzsaeure. Nach beendeter Zugabe ruehrt man noch 30 min bei Raumtemperatur weiter und dampft dann bei 30° Badtemperatur ein. Zur Entfernung von Restwassermengen wird mehrmals nach Zugabe von Alkohol eingedampft. Die letzten Reste an Loesungsmittel werden durch Anlegen von Hochvakuum entfernt. Man erhaelt so 40.4 g R-1-Chlor-2-hydroxy-3-mesyloxypropan. Dieses wird in 105 ml trockenem Ethylenglykol geloest. Nach Zugabe einer Loesung von 5.2 g Natrium in 130 ml trockenem Ethylenglykol wird noch 15 min bei Raumtemperatur geruehrt. Das entstandene R (-)-Epichlorhydrin wird sofort aus der Reaktionsloesung durch Anlegen von Hochvakuum (0.1-0.2 mm) bei Raumtemperatur abdestilliert. Zur Kondensation des R (-)-Epichlorhydrins wird der Kuehler mit einer Kuehlsole von -40°C bis -50°C beschickt. Der Auffangkolben wird ebenfalls auf diese Temperatur abgekuehlt.

Man erhaelt so 15.7 g R (-)-Epichlorhydrin. (Ausbeute: 78 %) $[\alpha]^{20}_D$ 33.8° (c = 1, Methanol)

**Beispiel 3**

**S (+)-4-(2,3-Epoxy-propoxy)-carbazol**

27.5 g 4-Hydroxy-carbazol werden in einer Mischung aus 150 ml 1 N Natronlauge und 70 ml Dimethylsulfoxid geloest. Hierzu gibt man bei Raumtemperatur 13.9 g R (-)-Epichlorhydrin und ruehrt 18 Stdn. bei Raumtemperatur weiter. Dann gibt man 280 ml Wasser zu, ruehrt noch 15 min und saugt ab. Der Filterrueckstand wird mit 0.1 N Natronlauge und Wasser gewaschen und anschließend in Methylenchlorid geloest. Die Methylenchloridloesung wird ueber Natriumsulfat getrocknet, mit Aktivkohle und Floridin behandelt und eingedampft. Der Rueckstand wird zur Reinigung zweimal aus Essigester umkristallisiert.

Ausbeute: 15.2 g Schmp: 163-164°C
$[\alpha]^{20}_D$: + 64.4° (c = 1; Pyridin)
Aus den Mutterlaugen wurden weitere 6.7 g Substanz isoliert.
Schmp: 163-164°C
$[\alpha]^{20}_D$: + 64.5° (c = 1; Pyridin)

**Beispiel 4**

**R (-)-4-(2,3-Epoxy-propoxy)-carbazol**

21.9 g 4-Hydroxy-carbazol werden in einer Mischung aus 120 ml 1 N Natronlauge und 40 ml Dimethylsulfoxid geloest. Hierzu tropft man bei Raumtemperatur eine Loesung von 18.2 g S (+)-3-Mesyloxy-1,2-epoxy-propan in 20 ml Dimethylsulfoxid. Man ruehrt 7 Stdn. bei Raumtemperatur, gibt 225 ml Wasser zu, ruehrt noch 15 min und saugt ab. Der Filterrueckstand wird mit 0.1 N Natronlauge und Wasser gewaschen und anschließend in Methylenchlorid geloest. Die Methylenchloridphase wird ueber Natriumsulfat getrocknet, mit Aktivkohle und Floridin behandelt und eingedampft. Der Rueckstand wird zur Reinigung zweimal aus Essigester umkristallisiert.

Ausbeute: 18.5 g Schmp: 162-163°C
$[\alpha]^{20}_D$: - 63.4° (c = 1; Pyridin)

**Beispiel 5**

**S (-)-[1-Carbazolyl-(4)-oxy]-3-isopropylamino-propanol-(2)-hydroacetat**

500 mg S (+)-4-(2,3-Epoxy-propoxy)-carbazol werden in 4 ml Methanol geloest und die Loesung nach Zugabe von 2.8 ml Isopropylamin 2 Stdn. auf 65°C erhitzt. Man dampft zu Trockene ein, wobei darauf zu achten ist, daß kein Isopropylamin mehr vorhanden ist, loest den Rueckstand heiß in 10 ml Essigester und versetzt die Loesung mit 0.24 ml Eisessig. Beim Abkuehlen kristallisiert das S (-)-Carazolol-Hydroacetat aus. Der Niederschlag wird abfiltriert, mit Essigester gewaschen und getrocknet.

Ausbeute: 410 mg Schmp: 158-160°C
$[\alpha]^{20}_D$: - 20.1° (c = 1; Eisessig)
optische Reinheit nach GC-Befund: 99.5 %

**Beispiel 6**

**R (+)-[1-Carbazolyl-(4)-oxy]-3-isopropylamino-propanol-(2)-hydroacetat**

18 g R (-)-4-(2,3-Epoxy-propoxy)-carbazol werden in 140 ml Methanol geloest und die Loesung nach Zugabe von 100 ml Isopropylamin 2 Stdn. auf 65°C erhitzt. Man dampft zur Trockene, trocknet noch 1 Std. im Hochvakuum nach zur Entfernung des restlichen Isopropylamins und loest den Rueckstand in 300 ml heißem Essigester. Die Essigesterloesung wird mit Floridin behandelt und nach dem Absaugen noch heiß mit 8.6 ml Eisessig versetzt. Man laeßt abkuehlen und saugt die ausgefallenen Kristalle ab. Zur weiteren Reinigung werden die Kristalle aus Essigester unter Zusatz von wenig Methanol umkristallisiert.

Ausbeute: 23 g Schmp: 158-160°C
$[\alpha]^{20}_D$: +20.2° (c = 1; Eisessig)
optische Reinheit: 98.6 %
chem. Reinheit: 99.97 %

**Beispiel 7**

**R(+)-[1-Carbazolyl-(4)-oxy]-3-[2-(2-methoxy-phenoxy)]-ethylamino-propanol-(2)**

5 g R(-)-4-(2,3-Epoxy-propoxy)-carbazol werden zusammen mit 6.9 g o-Methoxy-phenoxy-ethylamin in 35 ml Isopropanol 2 Stdn. am Rueckfluß erhitzt. Man dampft das Loesungsmittel ab und ruehrt den Rueckstand 2 Stdn. mit einer Mischung aus 115 ml Toluol, 35 ml Cyclohexan und 40 ml Essigester. Man saugt ab und kristallisiert den Rueckstand aus 150 ml Essigester um.

Ausbeute: 3.7 g Schmp. 121-123°C
$[\alpha]^{20}_D$: +18.4 (c = 1; Eisessig)

**Beispiel 8**

**S(-)-[1-Carbazolyl-(4)-oxy]-3-[2-(2-methoxy-phenoxy)]-ethylamino-propanol-(2)**

10 g S(+)-4-(2,3-Epoxy-propoxy)-carbazol werden zusammen mit 13.97 g o-Methoxy-phenoxy-ethylamin in 70 ml Isopropanol 2 Stdn. am Rueckfluß erhitzt. Man dampft das Loesungsmittel ab und ruehrt den Rueckstand 2 Stdn. mit einer Mischung aus 115 ml Toluol, 35 ml Cyclohexan und 40 ml Essigester. Man saugt ab und kristallisiert den Rueckstand aus 150 ml Essigester um.

Ausbeute: 7.2 g Schmp. 121-123°C
$[\alpha]^{20}_D$: -18.4 (c = 1; Eisessig)

## Patentansprüche

1. Verfahren zur Herstellung von R-Carbazol-Derivaten der Formel I

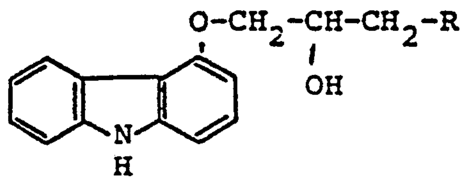

I,

in der
R eine Aminogruppe, die durch eine $C_1$-$C_6$-Alkylgruppe substituiert sein kann oder einen Rest

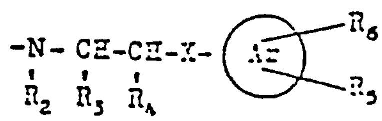

in dem
$R_2$ Wasserstoff, eine $C_1$-$C_6$-Alkylgruppe oder eine Benzyl-, Phenylethyl- oder Phenylpropylgruppe,
$R_3$ Wasserstoff oder eine $C_1$-$C_6$-Alkylgruppe,
$R_4$ Wasserstoff oder eine $C_1$-$C_6$-Alkylgruppe,
X einen Valenzstrich, eine-$CH_2$-Gruppe, ein Sauerstoffoder Schwefelatom,
Ar einen Phenyl-, Naphthyl-, Indanyl-, Tetrahydronaphtyl- oder Pyridyl-Rest und
$R_5$ und $R_6$, welche gleich oder verschieden sein können, jeweils Wasserstoff, Halogen, eine $C_1$-$C_6$-Alkylgruppe, eine Aminocarbonylgruppe, eine Hydroxygruppe, eine $C_1$-$C_6$-Alkoxygruppe, eine Benzyloxygruppe, eine $C_1$-$C_6$-Alkylmercapto-gruppe, eine $C_1$-$C_6$-Alkylsulfinylgruppe, eine $C_1$-$C_6$-Alkylsulfonylgruppe oder auch gemeinsam eine Methylendioxygruppe bedeuten,
darstellt,
sowie deren pharmakologisch verträgliche Salze, dadurch gekennzeichnet, daß man ein S-Epoxid-Derivat der Formel II

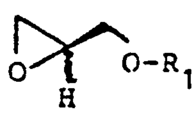

II,

in der $R_1$ ein substituiertes Sulfonsäure-Derivat bedeutet,
mit 4-Hydroxy-carbazol in Gegenwart eines organischen Lösungsmittels in alkalischem Medium umsetzt und das erhaltene
R-4-(2,3-Expoxy-propoxy)-carbazol
mit Ammoniak oder einem substituierten Amin der oben angegebenen Bedeutung RH umsetzt und anschließend die erhaltene Verbindung gewünschtenfalls in ein pharmakologisch verträgliches Salz überführt.

2. Verfahren nach Anspruch 1, dadurch gekennzeichnet, daß R ein Isopropylamin, tert. Butylamin- oder o-Methoxy-phenoxy-ethylamin-Rest und $R_1$ Mesyl bedeuten.

3. Verfahren zur Herstellung von S-Carbazol-Derivaten der Formel I

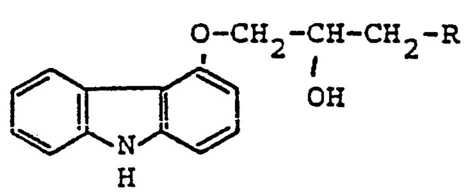

I,

in der
R eine Aminogruppe, die durch eine $C_1$-$C_6$-Alkylgruppe substituiert sein kann oder einen Rest

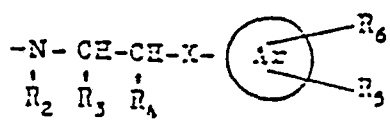

in dem
$R_2$ Wasserstoff, eine $C_1$-$C_6$-Alkylgruppe oder eine Benzyl-, Phenylethyl- oder Phenylpropylgruppe,
$R_3$ Wasserstoff oder eine $C_1$-$C_6$-Alkylgruppe,
$R_4$ Wasserstoff oder eine $C_1$-$C_6$-Alkylgruppe,
X einen Valenzstrich, eine -$CH_2$-Gruppe, ein Sauerstoffoder Schwefelatom,
Ar einen Phenyl-, Naphthyl-, Indanyl-, Tetrahydronaphthyl- oder Pyridyl-Rest und
$R_5$ und $R_6$, welche gleich oder verschieden sein können, jeweils Wasserstoff, Halogen, eine $C_1$-$C_6$-Alkylgruppe, eine Aminocarbonylgruppe, eine Hydroxygruppe, eine $C_1$-$C_6$-Alkoxygruppe, eine Benzyloxygruppe, eine $C_1$-$C_6$-Alkyl-mercaptogruppe, eine $C_1$-$C_6$-Alkylsulfinyl-gruppe, eine $C_1$-$C_6$-Alkylsulfonylgruppe oder auch gemeinsam eine Methylendioxygruppe bedeuten,
darstellt,
sowie deren pharmakologisch verträgliche Salze, dadurch gekennzeichnet, daß man
R-(-)Epichlorhydrin mit 4-Hydroxy-carbazol in Gegenwart eines organischen Lösungsmittels in alkalischem Medium umsetzt und das erhaltene
S-4-(2,3-Epoxy-propoxy)-carbazol
mit Ammoniak oder einem substituierten Amin der oben genannten Bedeutung RH umsetzt und anschließend die erhaltene Verbindung gewünschtenfalls in ein pharmakologisch verträgliches Salz überführt.

4. Verfahren nach Anspruch 3 dadurch gekennzeichnet, daß R ein Isoprofylamin, tert. Butylamin- oder o-Methoxyphenoxy-ethylamin-Rest bedeuten.

5. R(+)- und S(-)-Carbazolderivate der allgemeinen Formel I

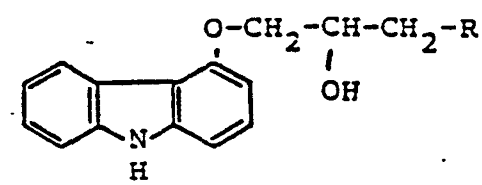
I,

in der
R einer der in den Ansprüchen 1 oder 2 genannten Aminogruppen
darstellt,
sowie deren pharmakologisch verträgliche Salze.

6. R(+)- und S(-)-[1-Carbazolyl-(4)-oxy]-3-[2-(2-methoxy-phenoxy)]-ethylamino-propanol-(2) sowie deren pharmakologisch verträgliche Salze.

7. R(-) und S(+)-4-(2,3-Epoxy-propoxy)-carbazol.

8. Arzneimittel enthaltend eine Verbindung gemäß Anspruch 5 sowie an sich bekannte pharmakologisch verträgliche Trägerstoffe.

9. Arzneimittel enthaltend eine Verbindung gemäß Anspruch 6 sowie an sich bekannte pharmakologisch verträgliche Trägerstoffe.

10. Arzneimittel enthaltend ein optisch reines Enantiomeres gemäß Anspruch 5, dadurch gekennzeichnet, daß neben üblichen pharmakologisch verträglichen Trägerstoffen ein adäquater Anteil des anderen Enantiomeren vorhanden ist, wobei das Enantiomeren-Verhältnis R: S von 50: 50 ausgeschlossen ist.

11. Arzneimittel enthaltend ein optisch reines Enantiomeres gemäß Anspruch 6, dadurch gekennzeichnet, daß neben üblichen pharmakologisch verträglichen Trägerstoffen ein adäquater Anteil des anderen Enantiomeren vorhanden ist, wobei das Enantiomeren-Verhältnis R: S von 50: 50 ausgeschlossen ist.

12. Verwendung von Verbindungen gemäß Anspruch 5 und 6 zur Herstellung von Arzneimitten.

13. Verwendung von Verbindungen gemäß Anspruch 7 zur Herstellung von Verbindungen der Formel I gemäß Anspruch 1 bzw. 3.

**Claims**

1. Process for the preparation of R-carbazole derivatives of the formula I

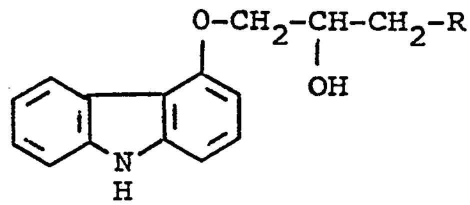

I

in which R represents an amino group which can be substituted by a $C_1$-$C_6$-alkyl group or a radical

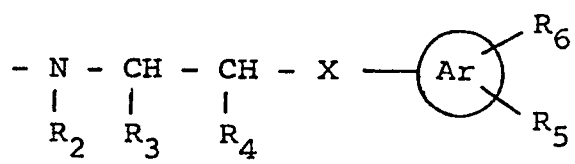

in which $R_2$ signifies hydrogen, a $C_1$-$C_6$ alkyl group or a benzyl, phenylethyl or phenylpropyl group, $R_3$ hydrogen or a $C_1$-$C_6$-alkyl group, $R_4$ hydrogen or a $C_1$-$C_6$-alkyl group, X a valency bond, a -$CH_2$- group, an oxygen or sulphur atom, Ar a phenyl, naphthyl, indanyl, tetrahydronaphthyl or pyridyl radical and $R_5$ and $R_6$, which can be the same or different, each represent hydrogen, halogen, a $C_1$-$C_6$-alkyl group, an aminocarbonyl group, a hydroxyl group, a $C_1$-$C_6$-alkoxy group, a benzyloxy group, a $C_1$-$C_6$-alkylmercapto group, a $C_1$-$C_6$-alkylsulphinyl group, a $C_1$-$C_6$-alkylsulphonyl group or also together a methylenedioxy group, as well as of their pharmacologically acceptable salts, characterised in that one reacts an S-epoxide derivative of the formula II

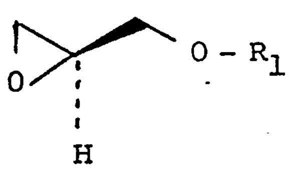

II,

in which $R_1$ signifies a substituted sulphonic acid derivative, with 4-hydroxycarbazole in the presence of an organic solvent in alkaline medium and reacts the R-4-(2,3-epoxypropoxy)-carbazole obtained with ammonia or a substituted amine of the above-given meaning RH and subsequently, if desired, converts the compound obtained into a pharmacologically acceptable salt.

2. Process according to claim 1, characterised in that R signifies an isopropylamine, tert.-butylamine or o-methoxyphenoxyethylamine radical and $R_1$ mesyl.

3. Process for the preparation of S-carbazole derivatives of the formula I

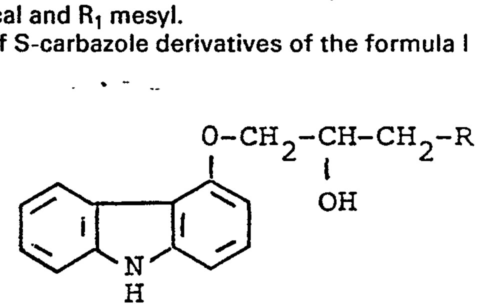

I,

in which R represents an amino group which can be substituted by a $C_1$-$C_6$-alkyl group or a radical

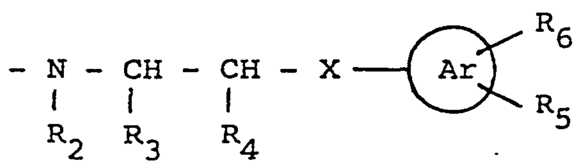

in which $R_2$ signifies hydrogen a $C_1$-$C_6$-alkyl group or a benzyl, phenylethyl or phenylpropyl group, $R_3$ hydrogen or a $C_1$-$C_6$-alkyl group, $R_4$ hydrogen or a $C_1$-$C_6$-alkyl group, X a valency bond, a -$CH_2$- group, an oxygen or sulphur atom, Ar a phenyl, naphthyl, indanyl, tetrahydronaphthyl or pyridyl radical and $R_5$ and $R_6$ which can be the same or different, each signify hydrogen, halogen, a $C_1$-$C_6$-alkyl group, an aminocarbonyl group, a hydroxyl group, a $C_1$-$C_6$-alkoxy group, a benzyloxy group, a $C_1$-$C_6$-alkylmercapto group, a $C_1$-$C_6$-alkylsulphinyl group, a $C_1$-$C_6$-alkylsulphonyl group or together also a methylenedioxy group, as well as of their pharmacologically acceptable salts, characterised in that one reacts R-(-)-epichlorhydrin with 4-hydroxycarbazole in the presence of an organic solvent in alkaline medium and reacts the S-4-(2,3-epoxypropoxy)-carbazole obtained with ammonia or a substituted amine of the above-mentioned meaning RH and subsequently, if desired, converts the compound obtained into a pharmacologically acceptable salt.

4. Process according to claim 3, characterised in that R signifies an isopropylamine, tert.-butylamine or o-methoxyphenoxyethylamine radical.

5. R(+)- and S(-)-carbazole derivatives of the general formula I

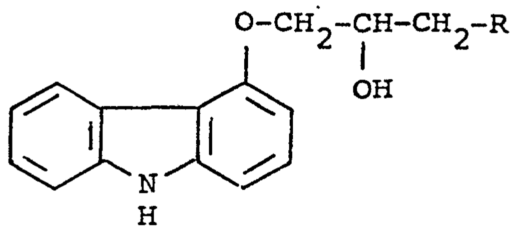

I

in which R represents one of the amino groups mentioned in claim 1 or 2, as well as their pharmacologically acceptable salts.

6. R(+)- and S(-)-[1-Carbazolyl-(4)-oxy]-3-[2-(2-methoxyphenoxy)]-ethylaminooropan-2-ol, as well as their pharmacologically acceptable salts.

7. R(-)- and S(+)-4-(2,3-epoxypropoxy)-carbazole.

8. Medicament containing a compound according to claim 5, as well as per se known pharmacologically acceptable carrier materials.

9. Medicament containing a compound according to claim 6, as well as per se known pharmacologically acceptable carrier materials.

10. Medicament containing an optically pure enantiomer according to claim 5, characterised in that, besides conventional pharmacologically acceptable carrier materials, an adequate proportion of the other enantiomer is present, whereby the enantiomer ratio R: S of 50: 50 is excluded.

11. Medicament containing an optically pure enantiomer according to claim 6, characterised in that, besides conventional pharmacologically acceptable carrier materials, an adequate proportion of the other enantiomer is present, whereby the enantiomer ratio R: S of 50: 50 is excluded.

12. Use of compounds according to claim 5 and 6 for the preparation of medicaments.

13. Use of compounds according to claim 7 for the preparation of compounds of the formula I according to claim 1 or 3.

**Revendications**

1. Procédé de préparation de dérivés de R-carbazole de la formule I:

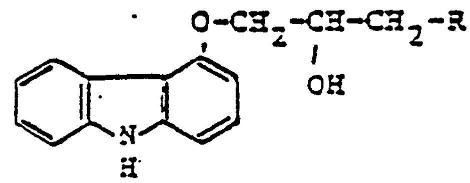

I,

dans laquelle
R représente un groupe amine qui peut être substitué par un groupe alkyle en $C_1$ à $C_6$, ou un radical

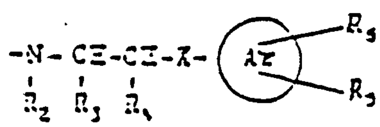

dans lequel:
$R_2$ représente l'hydrogène, un groupe alkyle en $C_1$ à $C_6$, ou un groupe benzyle, phényléthyle ou phénylpropyle;
$R_3$ l'hydrogène ou un groupe alkyle en $C_1$ à $C_6$,
$R_4$ l'hydrogène ou un groupe alkyle en $C_1$ à $C_6$,
X un trait de valence, un groupe $CH_2$, un atome d'oxygène ou de soufre,
Ar un radical phényle, naphtyle, indanyle, tétrahydronaphtyle ou pyridyle, et,
$R_5$ et $R_6$, qui peuvent être semblables ou différents, chacun l'hydrogène, un halogène, un groupe alkyle en $C_1$ à $C_6$, un groupe aminocarbonyle, un groupe hydroxyle, un groupe alcoxyle en $C_1$ à $C_6$, un groupe benzyloxyle, un groupe alkylmercapto en $C_1$ à $C_6$, un groupe alkylsulfinyle en $C_1$ à $C_6$, un groupe alkylsulfonyle en $C_1$ à $C_6$, ou encore, représentent ensemble un groupe méthylènedioxy,
ainsi que de leurs sels pharmacologiquement tolérables, caractérisé par le fait que l'on fait réagir:
un dérivé S-epoxy de la formule II:

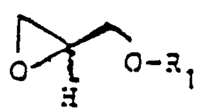

II,

dans laquelle $R_1$ représente un dérivé substitué d'acide sulfonique, sur le 4-hydroxycarbazole en présence d'un solvant organique, en milieu alcalin, et que l'on fait réagir le R-4-(2,3-époxypropoxy)-carbazole obtenu sur l'ammoniac ou une amine substituée de la signification RH indiquée plus haut, et qu'ensuite, si on le désire, on convertit le composé obtenu en un sel pharmacologiquement tolérable.

2. Procédé selon la revendication 1, caractérisé en ce que R représente un radical isopropylamine, tertiobutylamine ou o-méthoxyphénoxyéthylamine, et $R_1$ un radical mésyle.

3. Procédé de préparation de dérivés de S-carbazole de la formule I:

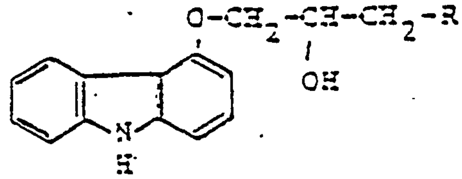

I,

dans laquelle
R représente un groupe amine qui peut être substitué par un groupe alkyle en $C_1$ à $C_6$, ou un radical

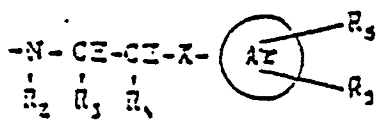

dans lequel:
$R_2$ représente l'hydrogène, un groupe alkyle en $C_1$ à $C_6$, ou un groupe bénzyle, phényléthyle ou phénylpropyle;
$R_3$ l'hydrogène ou un groupe alkyle en $C_1$ à $C_6$,
$R_4$ l'hydrogène ou un groupe alkyle en $C_1$ à $C_6$,
X un trait de valence, un groupe $CH_2$, un atome d'oxygène ou de soufre,
Ar un radical phényle, naphtyle, indanyle, tétrahydronaphtyle ou pyridyle, et,
$R_5$ et $R_6$, qui peuvent être semblables ou différents, chacun l'hydrogène, un halogène, un groupe alkyle en $C_1$ à $C_6$, un groupe aminocarbonyle, un groupe hydroxyle, un groupe alcoxyle en $C_1$ à $C_6$, un groupe benzyloxyle, un groupe alkylmercapto en $C_1$ à $C_6$, un groupe alkylsulfinyle en $C_1$ à $C_6$, un groupe alkylsulfonyle en $C_1$ à $C_6$, ou encore, représentent ensemble un groupe méthylènedioxy,
ainsi que de leurs sels pharmacologiquement tolérables, caractérisé par le fait que l'on fait réagir:
une R-(-)-épichlorhydrine sur le 4-hydroxycarbazole en présence d'un solvant organique, en milieu alcalin, et que l'on fait réagir le S-4-(2,3-époxypropoxy)-carbazole obtenu sur l'ammoniac ou une amine substituée de la signification RH indiquée plus haut, et qu'ensuite, si on le désire, on convertit le composé obtenu en un sel pharmacologiquement tolérable.

4. Procédé suivant la revendication 3, caractérisé en ce que R représente un radical isopropylamine, tertiobutylamine, ou o-méthoxyphénoxyéthylamine.

5. Dérivés de R(+)- et S(-)-carbazole de la formule générale I:

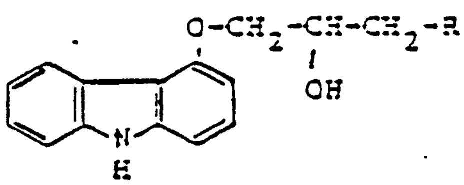

I,

dans laquelle:
R représente l'un des groupes amine indiqués aux revendications 1 ou 2, ainsi que leurs sels pharmacologiquement tolérables.

6. R(+)- et S(-)- [1-carbazolyl-(4)-oxy] -3-[2-(2-méthoxy-phénoxy)]-éthylamino-propanol-(2), ainsi que leurs sels pharmacologiquement tolérables.

7. R(-)- et S(+)-4-(2,3,-époxypropoxy)-carbazole.

8. Médicament contenant un composé selon la revendication 5, ainsi que des véhicules pharmacologiquement tolérables, en eux-mêmes connus.

9. Médicament contenant un composé selon la revendication 6, ainsi que des véhicules pharmacologiquement tolérables en eux-mêmes connus.

10. Médicament contenant un énantiomère optiquement pur selon la revendication 5, caractérisé par le fait qu'outre des véhicules usuels pharmacologiquement tolérables, est présente une proportion appropriée de l'autre énantiomère, le rapport des énantiomères R: S de 50: 50 étant exclu.

11. Médicament contenant un énantiomère optiquement pur selon la revendication 6, caractérisé par le fait qu'outre des véhicules usuels pharmacologiquement tolérables, est présente une proportion appropriée de l'autre énantiomère, le rapport des énantiomères R: S de 50: 50 étant exclu.

12. Utilisation de composés selon les revendications 5 et 6 pour la fabrication de médicaments.

13. Utilisation de composés selon la revendication 7 pour la préparation de composés de la formule I selon la revendication 1 ou 3.